# EUROPEAN PATENT SPECIFICATION

(11) **EP 2 505 187 B1**
(45) Date of publication and mention of the grant of the patent: **27.09.2017**
(21) Application number: 10832829.5
(22) Date of filing: 19.11.2010
(51) Int. Cl.: A61K 8/898, A61K 8/34, A61K 8/35, A61K 8/36, A61K 8/39, A61K 8/41, A61K 8/42, A61K 8/49, A61K 8/86, A61K 8/891, A61Q 5/00, A61Q 5/12, A61Q 5/02

(54) **HAIR COSMETIC**
KOSMETISCHES PRODUKT FÜR DAS HAAR
PRODUIT COSMÉTIQUE POUR LES CHEVEUX

(30) Priority: 25.11.2009 JP 2009267009; 16.11.2010 JP 2010255556
(43) Date of publication of application: 03.10.2012
(73) Proprietor: Kao Corporation, Chuo-Ku Tokyo 103-8210 (JP); Momentive Performance Materials Japan LLC, Tokyo 107-6109 (JP)
(72) Inventor: SUZUKI, Aya, Tokyo 131-8501 (JP); SUENAGA, Koji, Tokyo 107-6109 (JP); KAHARU, Takeshi, Tokyo 131-8501 (JP)
(74) Representative: Hoffmann Eitle
(86) International application number: PCT/JP2010/006806
(87) International publication number: WO 2011/064974

(56) References cited:
- WO-A1-03/084492
- JP-A- 2004 002 261
- JP-A- 2004 010 581
- JP-A- 2005 029 564
- JP-A- 2008 143 858
- JP-A- 2008 143 858
- JP-A- 2008 143 859
- JP-A- 2008 297 218
- JP-A- 2009 073 825
- JP-A- 2009 073 825
- JP-A- 2009 161 452
- Anonymous: "Silicone Hair Care Selection Guide", Dow Corning 'Toray' , 31 October 2008 (2008-10-31), page 6PP, XP002733805, Retrieved from the Internet: URL:http://www.kodawari-salon.com/image/y5 20.pdf [retrieved on 2014-12-15]

## Description

### TECHNICAL FIELD

The present invention relates to a hair cosmetic.

### BACKGROUND ART

Conventionally, a silicone compound is used for hair cosmetics as a component for imparting smoothness, moisturized feel, water repellency, shininess, and the like to hair. It is known that an amino-modified silicone has good adsorbability onto the hair surface and a particularly excellent effect in providing softness to hair due to an amino group in the molecule (PATENT DOCUMENTS 1, 2, 3, and so on). However, the above-mentioned amino-modified silicone has insufficient conditioning effects, such that it is difficult to run the fingers through the hair after drying. Furthermore, in recent years, the texture of an amino-modified silicone is losing popularity, and a lighter slippery feel, manageable feel, and freedom from stickiness have recently been desired.

Although various types of silicone wax have been proposed in order to impart a light slippery feel (for example, PATENT DOCUMENT 4), they cannot obtain a sufficient effect because of poor adsorbability onto hair and difficulty in remaining on the hair, and currently they are not in use.

In order to solve the above-mentioned problems, a specific modified silicone in which an amino group and an alkyl group are combined has been proposed, and it is described that the specific modified silicone can impart good slipperiness to both of wet hair and dry hair, and a light slippery feel and a manageable feel when finished (PATENT DOCUMENTS 5, 6).

### RELATED DOCUMENTS

### PATENT DOCUMENTS

[PATENT DOCUMENT 1] Japanese Patent Publication No. JP-A-09-110653
[PATENT DOCUMENT 2] Japanese Patent Publication No. JP-A-01-190619
[PATENT DOCUMENT 3] Japanese Patent Publication No. JP-A-08-217643
[PATENT DOCUMENT 4] Japanese Patent Publication No. JP-A-03-264510
[PATENT DOCUMENT 5] Japanese Patent Publication No. JP-A-2008-143858
[PATENT DOCUMENT 6] Japanese Patent Publication No. JP-A-2008-143859

JP 2009-073825 (A) describes a composition for hair, which contains a specific tertiary amine compound of a certain formula, an organic acid and/or an inorganic acid, and an aromatic alcohol. Fatty alcohols and silicones may optionally be included.

### DISCLOSURE OF THE INVENTION

However, the hair cosmetics disclosed in PATENT DOCUMENTS 5 and 6 have only a weak effect for damaged hair. Therefore, there is a desire for a technique that can exhibit the same conditioning effect and finished state for damaged hair as for healthy hair.

The present inventors have found that a hair cosmetic which is remarkably effective to meet the above-mentioned desire can be obtained by the use in combination of a specific side chain amino-modified organopolysiloxane having long chain alkyl at both ends, a cationic surfactant, a higher alcohol and a polydimethylsiloxane.

That is, the present invention provides a hair cosmetic containing the following components (A) to (D):
(A) 0.01 to 15 weight% of a side chain amino-modified organopolysiloxane having long chain alkyl at both ends represented by the following formula (1):

   (R¹R²₂)SiO-(R²₂SiO)ₓ-(R²R³SiO)_{y}-Si(R¹R²₂) (1)

   (wherein, in the above formula (1), R¹ represents a straight chain or branched alkyl group having 12 to 50 carbon atoms, R² represents a substituted or unsubstituted monovalent hydrocarbon group having 1 to 6 carbon atoms, R³ represents a 3-aminopropyl group or an N-(2-aminoethyl)-3-aminopropyl group, x is 1 to 2000, and y is an integer of 1 to 100);
(B) 0.1 to 15 weight% of one type or two or more types of cationic surfactants selected from tertiary amine salts;
(C) 0.1 to 20 weight% of a higher alcohol having 12 to 28 carbon atoms and one hydroxy group;
(D) 0.01 to 15 weight% of a polydimethylsiloxane; and
(E) water,
wherein a nitrogen atom content of the side chain amino-modified organopolysiloxane having long chain alkyl at both ends is 0.31 to 2 weight%.

In accordance with the present invention, there can be provided a hair cosmetic that can impart, even to damaged hair, light slipperiness when applying or during rinsing, light slipperiness after drying, softness, manageability, freedom from hair tangling, and freedom from stickiness.

### DESCRIPTION OF EMBODIMENTS

First, an outline of the present invention is explained.

The hair cosmetic of the present invention is a hair cosmetic containing:
(A) 0.01 to 15 weight% of a side chain amino-modified organopolysiloxane having long chain alkyl at both ends represented by the following formula (1):

   (R¹R²₂)SiO-(R²₂SiO)ₓ-(R²R³SiO)_{y}-Si(R¹R²₂) (1)

   (wherein, in the above formula (1), R¹ represents a straight chain or branched alkyl group having 12 to 50 carbon atoms, R² represents a substituted or unsubstituted monovalent hydrocarbon group having 1 to 6 carbon atoms, R³ represents a 3-aminopropyl group or an *N*-(2-aminoethyl)-3-aminopropyl group, x is 1 to 2000, and y is an integer of 1 to 100);
(B) 0.1 to 15 weight% in total of one type or two or more types of cationic surfactants selected from tertiary amine salts;
(C) 0.1 to 20 weight% of a higher alcohol having 12 to 28 carbon atoms and one hydroxy group;
(D) 0.01 to 15 weight% of a polydimethylsiloxane; and
(E) water,
wherein a nitrogen atom content of the side chain amino-modified organopolysiloxane having long chain alkyl at both ends is of 0.31 to 2 weight%.

### Component (A)

Component (A), the side chain amino-modified organopolysiloxane compound having long chain alkyl at both ends used in the present invention is represented by the formula (1) : (R¹R²₂)SiO-(R²₂SiO)ₓ-(R²R³SiO)_{y}-Si(R¹R²₂).

Here, R¹ is an alkyl group having 12 to 50 carbon atoms and may be straight chain or branched. A straight chain alkyl group is preferable from the viewpoint of suppressing tangling of hair after drying. Furthermore, a branched alkyl group is preferable from the viewpoint of fast-acting softness or an instant good shiny slipperiness when applying or during rinsing. R¹ is preferably an alkyl group having 14 to 40 carbon atoms, and more preferably an alkyl group having 14 to 30 carbon atoms. When R¹ is a straight chain alkyl group, an alkyl group having 16 to 18 carbon atoms is preferable. When R¹ is a branched alkyl group, an alkyl group having 16 to 30 carbon atoms is preferable, an alkyl group having 16 to 24 carbon atoms is more preferable, and an alkyl group having 18 to 24 carbon atoms is even more preferable.

Furthermore, the side chain amino-modified organopolysiloxane (A) having long chain alkyl at both ends is preferably a liquid (that is, 5 to 100,000 mPa·s) at 25°C from the viewpoint of imparting softness after drying and a light slippery feel at the same time.

R² is a substituted or unsubstituted monovalent hydrocarbon group having 1 to 6 carbon atoms; examples of the unsubstituted hydrocarbon group include a straight chain or branched alkyl group such as a methyl group, an ethyl group, a butyl group, or a hexyl group, a cycloalkyl group such as a cyclohexyl group, an alkoxy group such as a methoxy group, an ethoxy group, a propoxy group, or a butoxy group, an aryl group such as a phenyl group, a tolyl group, or a naphthyl group, an aralkyl group such as a benzyl group, a β-phenylethyl group, or a methylbenzyl group, and an alkenyl group such as a vinyl group or an allyl group. Examples of the substituted hydrocarbon group include a fluoroalkyl group such as a 3,3,3-trifluoropropyl group. In particular, an alkyl group and an aryl group are preferable, and a methyl group and a phenyl group are more preferable.

R³ is a 3-aminopropyl group or an *N*-(2-aminoethyl)-3-aminopropyl group.

In the formula (1) for Component (A), the plurality of R¹s may be identical groups or different groups, and the plurality of R²s may also be identical groups or different groups. Moreover, when there are a plurality of R³s, they may be identical groups or different groups. However, the R¹s are preferably identical groups from the viewpoint of ease of manufacturing.

The value of x is 1 or more from the viewpoint of slip properties during rinsing and slip properties after drying, and it is no more than 2000 from the viewpoint of the finished state after drying; it is preferably 1 to 1500, and more preferably 150 to 700. When R¹ in the formula (1) is a straight chain alkyl group, the value of x is preferably 200 to 400 from the viewpoint of softness after drying, and when R¹ in the formula (1) is a branched alkyl group, it is preferably 100 to 300 from the viewpoint of shiny slipperiness during application and rinsing and softness after drying.

The value of y is 1 or more from the viewpoint of adsorbability onto hair, it is no more than 100 from the viewpoint of preventing hair slipperiness from becoming heavy and attaining a finished state with light slipperiness, and it is preferably 1 to 50. Furthermore, when R¹ in the formula (1) is a straight chain alkyl group, the value of y is preferably 2 to 16 from the viewpoint of softness after drying, more preferably 2 to 8, and even more preferably 3 to 7. When R¹ in the formula (1) is a branched alkyl group, the value of y is preferably 1 to 16 from the viewpoint of shiny slipperiness during application and rinsing and softness after drying, more preferably 1 to 8, and even more preferably 1 to 3.

When R¹ in the formula (1) is a straight chain alkyl group, a composition for which the value of x being 200 to 400 is combined with the value of y being 3 to 7 is preferable from the viewpoint of softness after drying.

When R¹ in the formula (1) is a branched alkyl group, a composition to combine the value of x being defined as 100 to 300 with the value of y being defined as 1 to 3 is preferable from the viewpoint of shiny slipperiness during application and rinsing, and softness after drying.

Furthermore, it is preferable for the ratio of x and y to be in a specific range of y/x = 0.003 to 0.1 from the viewpoint of adsorbability onto hair, which gives hair after drying having light slipperiness and luster with manageability, freedom from hair tangling, and freedom from stickiness. It is more preferably in the range of 0.004 to 0.05.

In order to make y/x be in the above-mentioned specific range, the amount of amino group-containing silane or amino group-containing silicone compound and the amount of silicone monomer, which are starting materials, may be adjusted.

The nitrogen atom content of Component (A) is preferably 0.31 weight% or more from the viewpoint of reliably exhibiting a conditioning effect such as imparting slipperiness to damaged hair. Furthermore, the nitrogen atom content of Component (A) is preferably in the range of no more than 2 weight%, and more preferably in the range of 0.4 to 1.5 weight%, from the viewpoint of lightening the slippery feel in a wet state or a dry state. When R¹ in the formula (1) is a straight chain alkyl group, it is even more preferably in the range of 0.4 to 1 weight% from the viewpoint of softness after drying. When R¹ in the formula (1) is a branched alkyl group, it is even more preferably in the range of 0.4 to 0.8 weight% from the viewpoint of shiny slipperiness during application and rinsing and softness after drying.

The nitrogen atom content may be set in a desired range by adjusting the amount of amino group-containing silane or amino group-containing silicone compound.

The nitrogen atom content of Component (A) may be calculated as follows.

The nitrogen atom content is the content of nitrogen atoms contained in the side chain amino-modified organopolysiloxane having long chain alkyl at both ends and is measured by neutralization titration.

A certain amount (0.5 to 10 g) of sample is dissolved in about 10 times the amount of a mixed solution (1:1 [vol%]) of isopropyl alcohol and toluene. Potassium tetrabromophenolphthalein ethyl ester is added thereto as an indicator, and neutralization titration is carried out using an aqueous solution of perchloric acid. The content of nitrogen atoms in the side chain amino-modified organopolysiloxane compound having long chain alkyl at both ends is calculated from the amount of sample taken and the normality and amount consumed of the aqueous solution of perchloric acid.

Although PATENT DOCUMENT 6 discloses a side chain amino-modified organopolysiloxane compound having long chain alkyl at both ends, it is described that the slippery feel becomes heavy, when the nitrogen atom content exceeds 0.3 weight%. On the other hand, it has been found in the present invention that a light slipperiness when applying or during rinsing, that is, in a wet hair state, shiny slipperiness, a light slippery feel after drying, suppleness, manageability, freedom from hair tangling, freedom from stickiness, and luster can be imparted even to damaged hair, when the nitrogen atom content of a side chain amino-modified organopolysiloxane compound having long chain alkyl at both ends is 0.31 weight% or more in using specific Components (A) to (E).

The viscosity at 25°C of Component (A) is preferably 5 to 100,000 mPa·s, and more preferably 10 to 50,000 mPa·s. When it is no more than 100,000 mPa·s, the resistance of the hair cosmetic to uniformly spreading on the hair surface to thus cause nonuniform heavy slipperiness is suppressed. On the other hand, when it is 5 mPa·s or more, there is an effect of remaining on the hair and giving an actual feel of the finished state with slipperiness. A range of 50 to 9500 mPa·s is preferable, and a range of 500 to 4000 mPa·s is more preferable.

The viscosity of Component (A) is measured as follows.

The viscosity of the side chain amino-modified organopolysiloxane compound having long chain alkyl at both ends may be measured using a commercial rotational viscometer. Examples of the commercial viscometer include a Brookfield viscometer (Brookfield, USA) and a Vismetron viscometer (Shibaura Systems Co., Ltd.). Here, the viscosity at 25°C of the side chain amino-modified organopolysiloxane compound having long chain alkyl at both ends is measured using a Vismetron viscometer (Shibaura Systems Co., Ltd.).

The configuration of Component (A) when formulated into the hair cosmetic may be either an oil or emulsion.

The content of Component (A) in the hair cosmetic is preferably 0.01 weight% (hereinafter, also given simply as %) or more of the hair cosmetic of the present invention from the viewpoint of obtaining a good conditioning effect, and it is preferably no more than 15 weight% from the viewpoint of softness. Furthermore, it is more preferably 0.1 to 10 weight%, and even more preferably 0.1 to 5 weight%. It is preferably 0.1 to 2 weight% from the viewpoint of a finished state without stickiness. It is more preferably 0.1 to 0.8 weight%.

Component (A) as described above may be produced as follows.

A side chain amino-modified organopolysiloxane compound having long chain alkyl at both ends as Component (A) may be produced by an equilibrium reaction which is a typical production method for a silicone compound. That is, it is a method in which a cyclic siloxane such as octamethylcyclotetrasiloxane as a silicone monomer is equilibrated together with an end-blocking agent and an amino group-containing silane or amino group-containing silicone compound.

With regard to a catalyst used in the equilibrium reaction, any of an alkali catalyst such as sodium hydroxide, potassium hydroxide, or cesium hydroxide, a quaternary ammonium catalyst such as tetramethylammonium hydroxide, a quaternary phosphonium catalyst such as tetrabutylphosphonium hydroxide, or a silanolate of these compounds may be used.

With regard to the amount of catalyst added in the equilibrium reaction, it is used in a range of 0.1 to 1000 ppm. It is preferably 1 to 500 ppm. When it is less than 0.1 ppm the reaction does not progress sufficiently, and when it is an amount exceeding 1000 ppm an effect commensurate with that amount cannot be obtained.

The reaction temperature in the equilibrium reaction may be any temperature as long as the reaction progresses, and it is usually carried out in the range of 50 to 200°C, and the reaction temperature is appropriately selected according to the catalyst used.

Since low molecular weight siloxanes are formed by the equilibrium reaction, these siloxane components may be removed as necessary by distillation under reduced or atmospheric pressure.

A side chain amino-modified organopolysiloxane compound (A) having long chain alkyl at both ends may thus be produced.

### Component (B)

As the cationic surfactant, which is Component (B), a tertiary amine compound represented by the formula (8) can be cited.

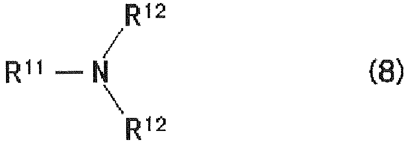

In the above formula (8), R¹¹ represents a straight chain or branched chain alkyl group, alkenyl group, or aliphatic acyloxy (polyethoxy) ethyl group that has 8 to 35 carbon atoms in total and may be divided by a functional group represented by -OCO- or -COO- or substituted with -OH, R¹² represents an alkyl group or hydroxyalkyl group having 1 to 22 carbon atoms or a polyoxyethylene group having a total number of moles added of no more than 10, and the 2 R¹²s may be identical to or different from each other.

As the tertiary amine compound represented by the formula (8), a salt of a tertiary amine formed by an organic acid and/or an inorganic acid may be used, or a salt may be formed in a composition while adjusting the pH by formulating an acid into the hair cosmetic of the present invention. Examples of such an acid include an acid having a short-chain alkyl group such as an alkylphosphoric acid, an alkylsulfonic acid, or an alkylsulfuric acid; an acidic amino acid such as L-glutamic acid or L-aspartic acid; pyroglutamic acid; an aromatic acid such as benzoic acid or p-toluenesulfonic acid; a hydroxy acid such as glycolic acid, lactic acid, glyceric acid, gluconic acid, pantothenic acid, malic acid, tartaric acid, or citric acid; and phosphoric acid, hydrochloric acid, acetic acid, succinic acid, and so on. In particular, an organic acid is preferable from the viewpoint of exhibiting moisturizing and softening effects for hair, an acidic amino acid, pyroglutamic acid, and hydroxy acid are more preferable, and a hydroxy acid is even more preferable.

Specific examples of the cationic surfactant as Component (B) include at least one of the following tertiary amine compounds (i) to (iii) (or salts thereof).

(i) Hydroxy ether alkylamine (or salt thereof)

For example, a compound represented by the following formula (12) and a salt thereof can be exemplified.

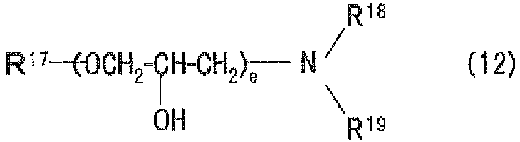

In the formula (12), R¹⁷ represents a straight chain or branched chain alkyl group or alkenyl group having 6 to 24 carbon atoms, and R¹⁸ and R¹⁹ may be identical to or different from each other and represent an alkyl group having 1 to 6 carbon atoms or -(AO)_{f}H (A represents an alkylene group having 2 to 4 carbon atoms, f represents 1 to 6, and the f of As may be identical to or different from each other and may be in any order). e represents 1 to 5.

Specific examples include hexadecyloxy(2-hydroxypropyl)dimethylamine and a salt thereof and octadecyloxy(2-hydroxypropyl)dimethylamine and a salt thereof.

### (ii) Ether amine (or salt thereof)

For example, a compound represented by the following formula (13) or a salt thereof can be cited.

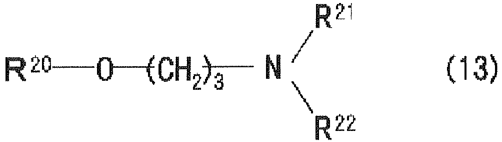

In the above formula (13), R²⁰ represents a straight chain or branched chain alkyl group or alkenyl group having 6 to 24 carbon atoms, and R²¹ and R²² are identical to or different from each other and represent an alkyl group having 1 to 6 carbon atoms or -(AO)gH (A represents an alkylene group having 2 to 4 carbon atoms, g represents 1 to 6, and the g As may be identical to or different from each other and may be in any order).

Specific examples include *N,N*-dimethyl-3-hexadecyloxypropylamine and a salt thereof and *N,N*-dimethyl-3-octadecyloxypropylamine and a salt thereof.

### (iii) Alkylamidoamine (or salt thereof)

For example, a compound represented by the following formula (14) or a salt thereof can be exemplified.

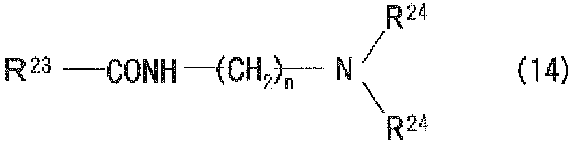

In the above formula (14), R²³ represents an aliphatic hydrocarbon group having 11 to 23 carbon atoms, the R²⁴s are identical to or different from each other and represent a hydrogen atom or an alkyl group having 1 to 4 carbon atoms, and n represents 2 to 4.

Specific examples include N-(3-(dimethylamino)propyl)docosanamide and a salt thereof and N-(3-dimethylamino)propyl)stearamide and a salt thereof.

As a preferred cationic surfactant selected from the above-mentioned tertiary amine compounds, (ii) an ether amine (or a salt thereof) or (iii) an alkylamidoamine (or a salt thereof) is preferable from the viewpoint of smoothness when applying and during rinsing.

In particular, (ii) an ether amine (or a salt thereof) is more preferable, and *N,N*-dimethyl-3-hexadecyloxypropylamine or a salt thereof and *N,N*-dimethyl-3-octadecyloxypropylamine or a salt thereof are even more preferable.

With regard to the cationic surfactant selected from the tertiary amines of Component (B), one type or 2 or more types in combination may be used. The content of Component (B) is preferably 0.1 to 15 weight% of the hair cosmetic of the present invention from the viewpoint of smoothness when applying and during rinsing, ease of running the fingers through, and imparting luster. It is more preferably 0.2 to 10 weight%, and even more preferably 0.5 to 5 weight%.

### Component (C)

As the higher alcohol having one hydroxy group, which is Component (C), one having an alkyl group having 12 to 28 carbon atoms is preferable, and one having an alkyl group having 16 to 24 is more preferable, and one having an alkyl group having 18 to 22 carbon atoms is even more preferable. This alkyl group preferably is a straight chain alkyl group. Specific examples include cetyl alcohol, stearyl alcohol, arachidyl alcohol, and behenyl alcohol. Stearyl alcohol and behenyl alcohol are more preferable.

The higher alcohol contains one hydroxy group and does not contain two or more hydroxy groups.

With regard to the higher alcohol having one hydroxy group as Component (C), one type or two or more types in combination may be used, and the content thereof is preferably 0.1 to 20 weight% of the hair cosmetic of the present invention; it is more preferably 0.5 to 15 weight%, and even more preferably 1 to 10 weight% from the viewpoint of smoothness, ease of application, and improvement of stability and so on.

### Component (D)

The polydimethylsiloxane as Component (D) is preferably one represented by the formula (I).

R⁵(CH₃)₂SiO-[(CH₃)₂SiO]ₐ-Si(CH₃)₂R⁵ (I)

In the formula (I), R⁵ represents a methyl group or a hydroxy group, and a represents 10 to 20000. When R⁵ is a methyl group, a is preferably 500 to 10000, and more preferably 1000 to 5000.

Various types of polydimethylsiloxane may be used, and examples of commercial products include DOW CORNING TORAY BY11-026 and BY22-060 (Dow Corning Toray), and KF-9008 and KF-9013 (Shin-Etsu Chemical Co., Ltd.).

They are specifically represented by the following formula.

(CH₃)₃SiO-[(CH₃)₂SiO]ₐ-Si(CH₃)₃

(a represents 100 to 20000.)

With regard to the polydimethylsiloxane, a solution or a dispersion thereof in a liquid oil (for example, a low degree of polymerization polydimethylsiloxane oil, a liquid silicone oil such as a cyclic silicone, or a liquid hydrocarbon oil such as an isoparaffin) may be used.

The content of the polydimethylsiloxane, which is Component (D), is preferably 0.01 weight% or more but no more than 15 weight% of the hair cosmetic of the present invention from the viewpoint of imparting slipperiness to hair. 0.05 to 15 weight% is more preferable, and 0.1 to 10 weight% is even more preferable from the viewpoint of a good conditioning effect and improvement of stability, etc. 0.1 to 0.8 weight% is yet more preferable.

The weight ratio of Component (A) and Component (D) is preferably (A)/(D) = 0.01 to 2. 0.02 to 1 is more preferable. It is even more preferably 0.05 to 0.3 from the viewpoint of stickiness. When a small amount of Component (A) is contained relative to the polydimethylsiloxane, which is Component (D), light slipperiness when applying or during rinsing, shiny slipperiness after drying, suppleness, and manageability can be imparted even to damaged hair, and tangling of hair and stickiness can be suppressed. Setting the weight ratio of Component (A) and Component (D) in the above-mentioned range is preferable in terms of suppleness after drying when R¹ of Component (A) represented by the formula (1) is a straight chain alkyl group, and is preferable in terms of shiny slipperiness during application and rinsing and suppleness after drying when R¹ of Component (A) represented by the formula (1) is a branched alkyl group.

With regard to the hair cosmetic of the present invention, either a production method in which Components (B) and (D) are separately added to a composition containing Components (A) and (C), other formulation Components and water or a production method in which a silicone phase in which Components (B) and (D) have been dissolved in each other in advance is added may be employed, and the production method in which Components (B) and (D) have been dissolved in each other in advance is more preferable. This enables light slipperiness when applying or during rinsing, light slipperiness after drying, suppleness, and freedom from stickiness to be imparted.

The content of water as Component (E) is preferably 35 weight% or more but no more than 99 weight% of the hair cosmetic of the present invention. It is more preferably 45 weight% or more but no more than 95 weight%.

Furthermore, the hair cosmetic of the present invention may contain a specific organic solvent (F). As the specific organic solvent, specifically, at least one organic solvent selected from the following (f1) to (f6) can be used. (f1): a compound represented by the following formula (2):

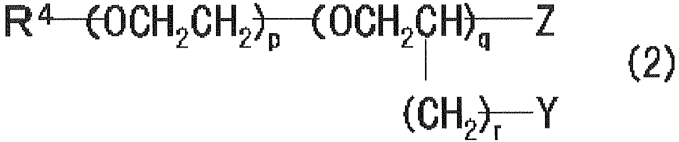

In the above formula (2), R⁴ represents a hydrogen atom, an alkyl group having 1 to 4 carbon atoms, or the group R²⁶-Ph-R²⁷ (R²⁶: hydrogen atom, methyl group or methoxy group, R²⁷: a direct bond or a saturated or unsaturated divalent hydrocarbon group having 1 to 3 carbon atoms, Ph: phenylene group), Y and Z independently represent a hydrogen atom or a hydroxy group, and p, q, and r independently represent an integer of 0 to 5. When p = q = 0, R⁴ is neither a hydrogen atom nor the group R²⁶-Ph-R²⁷ and Z is a hydroxy group.
Here, (f1) does not include Component (C) (a higher alcohol having 12 to 28 carbon atoms and one hydroxy group).
(f2): an *N*-alkylpyrrolidone in which an alkyl group having 1 to 18 carbon atoms is bonded to the nitrogen atom
(f3): an alkylene carbonate having an alkylene group having 2 to 4 carbon atoms
(f4): a polypropylene glycol having a molecular weight of 100 to 5000
(f5): a lactone or cyclic ketone represented by the following formula (3), (4), or (5):

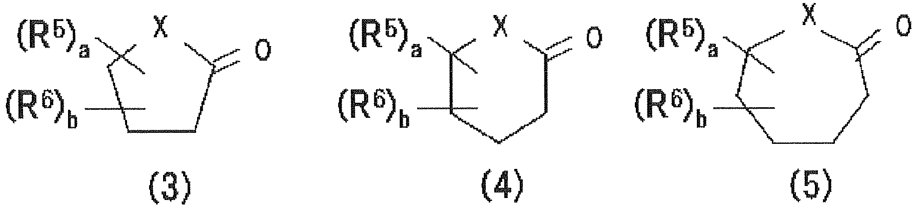

In the above formulae (3) to (5), X represents a methylene group or an oxygen atom, R⁵ and R⁶ represent mutually different substituents, and a and b independently represent 0 or 1.

(f6): a diol represented by the following formula (6):

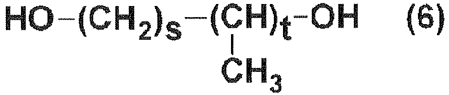

In the above formula (6), s and t independently represent an integer of 0 to 6. When s = 0, t is 1 or more. When t = 0, s is 1 or more.

In the present invention, among organic solvents as Component (F), examples of (f1) include ethanol, 1-propanol, 2-propanol, butanol, isobutanol, ethylene glycol, propylene glycol, benzyl alcohol, cinnamyl alcohol, phenethyl alcohol, *p*-anisyl alcohol, *p*-methylbenzyl alcohol, phenoxyethanol, 2-benzyloxyethanol, methyl carbitol, ethyl carbitol, propyl carbitol, butyl carbitol, triethylene glycol monoethyl ether, triethylene glycol monobutyl ether, and glycerol.

Examples of (f2) include *N*-methylpyrrolidone, *N-*octylpyrrolidone, and *N*-laurylpyrrolidone.

Examples of (f3) include ethylene carbonate and propylene carbonate.

With regard to the polypropylene glycol (f4), one having a molecular weight of 100 to 1000 is preferable from the viewpoint of a smooth feel and softness when rinsing after application to hair.

With regard to (f5), R⁵ and R⁶ in the above formulae (3) to (5) include preferably straight chain, branched chain, or cyclic alkyl groups, hydroxy groups, sulfonic acid groups, phosphoric acid groups, carboxy groups, phenyl groups, sulfoalkyl groups, alkyl phosphate groups, or carboxyalkyl groups. They are more preferably straight chain or branched chain alkyl groups having 1 to 6 carbon atoms, such as for example methyl groups, ethyl groups, propyl groups, isopropyl groups, or butyl groups, in which substitution is at the γ position in the case of a γ-lactone and the δ position in the case of a δ-lactone (that is, methylene adjacent to heterooxygen atom).

Furthermore, when it is desired to increase the water solubility of compounds represented by the above formulae (3) to (5), it is preferable for R⁵ or R⁶ to be an acidic group such as a sulfonic acid group, a phosphoric acid group, or a carboxy group, or an alkyl group substituted therewith.

Among (f5), examples of the lactone include γ-butyrolactone, γ-caprolactone, γ-valerolactone, δ-valerolactone, δ-caprolactone, and δ-heptanolactone. γ-Lactones are preferable from the viewpoint of stability of the lactone, and γ-butyrolactone and γ-caprolactone are more preferable.

Among (f5), examples of the cyclic ketone include cyclopentanone, cyclohexanone, cycloheptanone, and 4-methylcycloheptanone.

As (f6), propylene glycol, 1,3-butanediol, 1,5-hexanediol, and the like are preferable.

As Component (F), benzyl alcohol, 2-benzyloxyethanol, propylene carbonate, propylene glycol, dipropylene glycol, and 1,3-butanediol are more preferable from the viewpoint of a finished state such as luster or manageability.

With regard to Component (F), two or more types may be used in combination. The content thereof is preferably 0.01 weight% or more and 50 weight% or less from the viewpoint of a finished state such as luster or manageability of the hair cosmetic of the present invention, more preferably 0.1 to 35 weight%, and even more preferably 0.5 to 10 weight%.

The hair cosmetic of the present invention may further contain a specific organic acid (G).

Such an organic acid is preferably an organic acid having no more than 10 carbon atoms, and examples thereof include an acid having a short-chain alkyl group having no more than 10 carbon atoms such as an alkylphosphoric acid, an alkylsulfonic acid, or an alkylsulfuric acid, an acidic amino acid such as glutamic acid or aspartic acid, an aromatic acid such as benzoic acid or p-toluenesulfonic acid, a hydroxy acid, and a dicarboxylic acid.

Specific examples of the hydroxy acid include hydroxymonocarboxylic acids such as glycolic acid, lactic acid, glyceric acid, gluconic acid, and pantothenic acid, hydroxydicarboxylic acids such as malic acid and tartaric acid, and hydroxytricarboxylic acids such as citric acid.

Specific examples of the dicarboxylic acid include oxalic acid, malonic acid, maleic acid, succinic acid, and glutaric acid.

A hydroxymonocarboxylic acid, a hydroxydicarboxylic acid, a dicarboxylic acid, and an acidic amino acid are preferable in terms of an effect in moisturizing and softening hair. Glycolic acid, lactic acid, malic acid, tartaric acid, maleic acid, glutamic acid, and aspartic acid are more preferable. Glycolic acid, lactic acid, malic acid, maleic acid, and glutamic acid are even more preferable. It is yet more preferable for at least one or more kinds of organic acid selected from the group consisting of malic acid, lactic acid, glutamic acid, and glycolic acid to be contained. In the present invention, glutamic acid may be formulated into the hair cosmetic as an impurity in pyrrolidonecarboxylic acid.

With regard to these Components (G), one type on its own or two or more types in combination may be used. The content of Component (G) is preferably 0.1 to 5 weight% of the hair cosmetic of the present invention from the viewpoint of improvement of a finished feel of hair such as luster or manageability. It is more preferably 0.2 to 3 weight%, and even more preferably 0.5 to 2 weight%.

The hair cosmetic of the present invention preferably has a pH (diluted to 20 times its weight with water, 25°C) when applied to the hair of 2 or more and 5 or less, more preferably pH 2.5 to 4, and even more preferably pH 3 to 4 from the viewpoint of imparting luster, softness, manageability, and suppleness to the hair.

The hair cosmetic of the present invention may contain a conditioning agent selected from silicones and oils other than Components (A) and (D) in order to further improve the texture given to the hair.

Examples of silicones other than Components (A) and (D) include those below.

### Silicones-1: Amino-modified silicone

Various types of amino-modified silicone may be used, and one having an average molecular weight of about 3000 to 100000, described in CTFA Dictionary (Cosmetic Ingredient Dictionary, USA) Third Edition under the name of Amodimethicone (Amodimethicone), is more preferable. This amino-modified silicone is preferably used as an aqueous emulsion, and SM8704C [Dow Corning Toray], DC 929 [Dow Corning Corporation], and the like can be exemplified as a commercial product.

As other amino-modified silicones, examples include a compound represented by the formula (15), and as a commercial product, '8500 Conditioning Agent' by Dow Corning Corporation (CAS No. 237753-63-8) can be exemplified.

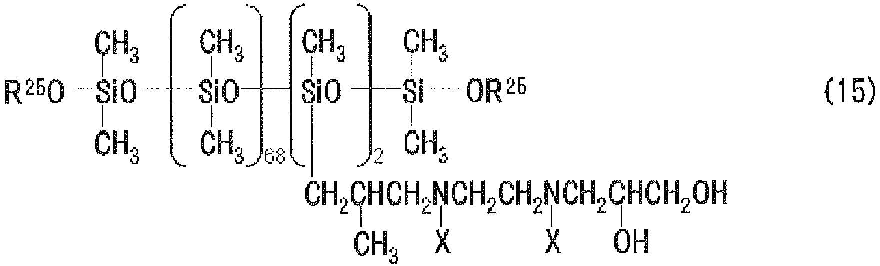

In the above formula (15), R²⁵ represents a straight chain or branched chain alkyl group having 13 to 15 carbon atoms, and 75% of the Xs represent the group -CH₂CH(OH)CH₂OH and 25% of the Xs represent a hydrogen atom.

Preferred examples of an amino-modified polysiloxane-polyoxyalkylene block copolymer include those represented by the following formula (16).

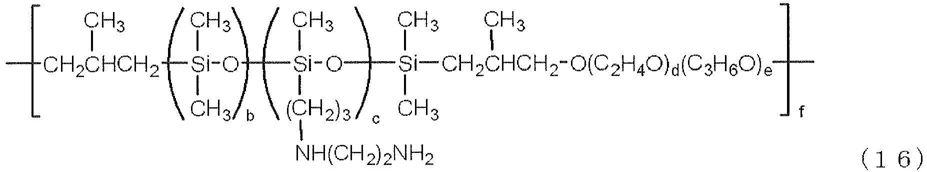

In the above formula (16), b represents an integer of 2 or more, c represents an integer of 1 or more, d represents an integer of 4 or more, e represents an integer of 0 to 30, and f represents an integer of 2 or more.

In the above formula (16), it is preferable that b represents 2 to 1,000, c represents 1 to 50, d represents 4 to 200, and f represents 2 to 100. Furthermore, - O(C₂H₄O)_{d}(C₃H₆O)ₑ- may be any of a block copolymer and a random copolymer. As commercial products, FZ-3789 and Silicone SS-3588 from Dow Corning Toray can be exemplified.

### Silicones-2: Other silicones

Other than the above-mentioned silicones, a polyether-modified silicone, a methyl phenyl polysiloxane, a fatty acid-modified silicone, an alcohol-modified silicone, an alkoxy-modified silicone, an epoxy-modified silicone, a fluorine-modified silicone, a cyclic silicone, an alkyl-modified silicone, and the like can be exemplified.

With regard to these silicones, two or more types thereof may be used in combination, and the content thereof is preferably 0.01 to 5 weight% of the hair cosmetic of the present invention, more preferably 0.05 to 1 weight%, and even more preferably 0.1 to 0.5 weight%, from the viewpoint of imparting hair smoothness, ease of running the fingers through the hair, and luster.

The oil referred to here means an oily substance other than a silicone, and examples thereof include hydrocarbons such as squalene, squalane, liquid paraffin, liquid isoparaffin, and cycloparaffin; glycerides such as castor oil, cocoa butter, mink oil, avocado oil, and olive oil; waxes such as beeswax, spermaceti, lanolin, and carnauba wax; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, coconut oil fatty acid, isostearic acid, and isopalmitic acid, and others such as isostearyl glyceryl ether and polyoxypropylene butyl ether. Among them, an ester is preferable, and hexadecyl 2-ethylhexanoate, isononyl isononanoate, isopropyl palmitate, and the like are more preferable. With regard to these oils, two or more types thereof may be used in combination, and the content thereof is preferably 0.01 to 15 weight% of the hair cosmetic of the present invention, more preferably 0.05 to 10 weight%, and even more preferably 0.1 to 7 weight%.

The hair cosmetic of the present invention may contain, in addition to the above-mentioned components, a component that is normally used in a hair cosmetic, according to the intended application. Examples of such components include a moisturizing agent such as sorbitol or panthenol; a colorant such as a dye or a pigment; a viscosity adjusting agent such as hydroxyethylcellulose, methylcellulose, polyethylene glycol, polyvinyl alcohol, or a clay mineral; a pH adjusting agent such as potassium hydroxide, sodium hydroxide, or citric acid; a plant extract; a pearlescent agent; a fragrance; a coloring agent; a UV absorbing agent; an antioxidant; and other components described in the Encyclopedia of Shampoo Ingredients [ENCYCLOPEDIA OF SHAMPOO INGREDIENTS (MICELLE PRESS)].

The hair cosmetic of the present invention may be made into a desired form such as an aqueous solution, an ethanol solution, an emulsion, a suspension, a gel, crystals, a solid, or an aerosol, and may be applied to for example a hair rinse, a hair treatment, hair pack, hair cream, conditioning mousse, hair mousse, a hair spray, a leave-on treatment, and the like. A form that is washed away during use such as a hair rinse, a hair conditioner, or a hair treatment is more suitable.

In accordance with the hair cosmetic of the present invention, light slipperiness and shiny slipperiness when hair is wet and a light slippery feel, manageable feel, freedom from hair tangling, freedom from stickiness, and luster after drying can be imparted not only to healthy hair but also even to damaged hair.

### EXAMPLES

In the examples below % means weight% unless otherwise specified.

R¹, R², R³, x, y, the nitrogen atom content (weight %), and the viscosity of side chain amino-modified organopolysiloxanes (A) having long chain alkyl at both ends (components (a1 to a4), formula (1))

(R¹R²₂)SiO-(R²₂SiO)ₓ-(R²R³SiO)_{y}-Si(R¹R²₂) (1)

[in the above formula (1), R¹ represents a straight chain or branched alkyl group having 12 to 50 carbon atoms, R² represents a substituted or unsubstituted monovalent hydrocarbon group having 1 to 6 carbon atoms, R³ represents a 3-aminopropyl group or an *N*-(2-aminoethyl)-3-aminopropyl group, x is 1 to 2000, and y is an integer of 1 to 100] used in the Examples and Comparative Examples below are summarized in Table 1 and Table 2.

The R¹s of all of the side chain amino-modified organopolysiloxanes (a1) to (a4) having long chain alkyl at both ends are straight chain, and the R¹s of (a5) and (a6) are branched.

The methods for producing these side chain amino-modified organopolysiloxanes having long chain alkyl at both ends were as follows.

### Side chain amino-modified organopolysiloxane (a1) having long chain alkyl at both ends

First, an alkyl-modified polyorganosiloxane represented by the formula:

R^{A}(CH₃)₂-SiO-((CH₃)₂SiO)₂₀-Si(CH₃)₂R^{A} (17)

[in the above formula (17), R^{A} is a straight chain alkyl group having 16 to 18 carbon atoms]
was synthesized.

Specifically, 100 parts of a mixture of 1-hexadecene and 1-octadecene (Linealene 168, Idemitsu Kosan Co., Ltd.) and 0.2 parts of a 1% isopropanol solution of chloroplatinic acid were heated to 70°C while stirring. Subsequently, 300 parts of a polyorganosiloxane having hydrogen at both ends represented by the formula (II) below was added dropwise at 70°C to 80°C. After the dropwise addition was completed, stirring was carried out at 80°C for 1 hour, and the reaction was ended on confirming that a peak due to Si-H in the IR spectrum had disappeared.

### Formula (II): H(CH₃)₂-SiO-((CH₃)₂SiO)₂₀-Si(CH₃)₂H

An alkyl-modified polyorganosiloxane having a viscosity of 0.030 Pa·s (measured with No. 1 rotor at 60 rpm) was thus obtained.

71 parts of the alkyl-modified polyorganosiloxane thus obtained, 633 parts of octamethylcyclotetrasiloxane, and 46 parts of *N*-(2-aminoethyl)-3-aminopropylmethyldimethoxysilane were heated to 90°C while stirring. Subsequently, a 27% aqueous solution of tetramethylammonium hydroxide was added so that the effective component was 90 ppm, and an equilibrium reaction was carried out at 90°C for 3 hours. Finally, low molecular weight siloxane was removed by distillation under reduced pressure, thus giving side chain amino-modified organopolysiloxane (a1) having long chain alkyl at both ends with a nitrogen atom content of 0.96% and a viscosity of 2,100 mPa·s (measured with No. 4 rotor at 60 rpm).

### Side chain amino-modified organopolysiloxane (a2) having long chain alkyl at both ends:

Side chain amino-modified organopolysiloxane (a2) having long chain alkyl at both ends with a nitrogen atom content of 0.62% and a viscosity of 3,500 mPa·s (measured with No. 4 rotor at 60 rpm) was obtained in the same manner as in the production method for (a1) except that the alkyl-modified polyorganosiloxane represented by the formula (17) was changed to 50 parts, octamethylcyclotetrasiloxane was changed to 669 parts, and *N*-(2-aminoethyl)-3-aminopropylme-thyldimethoxysilane was changed to 32 parts.

### Side chain amino-modified organopolysiloxane (a3) having long chain alkyl at both ends:

Side chain amino-modified organopolysiloxane (a3) having long chain alkyl at both ends with a nitrogen atom content of 0.22% and a viscosity of 19,000 mPa·s (measured with No. 4 rotor at 6 rpm) was obtained in the same manner as in the production method for (a1) except that the alkyl-modified polyorganosiloxane represented by the formula (17) was changed to 32 parts, octamethylcyclotetrasiloxane was changed to 707 parts, and *N*-(2-aminoethyl)-3-aminopropylmethyldimethoxysilane was changed to 11 parts.

### Side chain amino-modified organopolysiloxane (a7) having long chain alkyl at both ends:

Side chain amino-modified organopolysiloxane (a7) having long chain alkyl at both ends with a nitrogen atom content of 0.49% and a viscosity of 1,200 mPa·s (measured with No. 3 rotor at 30 rpm) was obtained in the same manner as in the production method for (a1) except that the alkyl-modified polyorganosiloxane represented by the formula (17) was changed to 74 parts, octamethylcyclotetrasiloxane was changed to 651 parts, and *N*-(2-aminoethyl)-3-aminopropylmethyldimethoxysilane was changed to 25 parts.

### Side chain amino-modified organopolysiloxane (a4) having long chain alkyl at both ends:

Side chain amino-modified organopolysiloxane (a4) having long chain alkyl at both ends with a nitrogen atom content of 0.20% and a viscosity of 10,000 mPa·s (measured with No. 3 rotor at 60 rpm) was obtained in the same manner as in the production method for (a1) except that an alkyl-modified polyorganosiloxane represented by:

Formula: R^{B}(CH₃)₂-SiO-((CH₃)₂SiO)₂₀-Si(CH₃)₂R^{B} (18)

(in the Formula, R^{B} is a straight chain alkyl group having 20 to 28 carbon atoms) was 35 parts, octamethylcyclotetrasiloxane was changed to 705 parts, and *N*-(2-aminoethyl)-3-aminopropylmethyldimethoxysilane was changed to 11 parts.

### Side chain amino-modified organopolysiloxane (a5) having long chain alkyl at both ends:

Side chain amino-modified organopolysiloxane (a5) having long chain alkyl at both ends with a nitrogen atom content of 0.40% and a viscosity of 700 mPa·s (measured with No. 3 rotor at 60 rpm) was obtained in the same manner as in the production method for (a1) except that an alkyl-modified polyorganosiloxane represented by:

Formula: R^{C}(CH₃)₂-SiO-((CH₃)₂SiO)₂₀-Si(CH₃)₂R^{C} (19)

(in the Formula, R^{C} is an alkyl group shown by the structure (Formula (20)) below) was 102 parts, octamethylcyclotetrasiloxane was changed to 628 parts, and N-(2-aminoethyl)-3-aminopropylmethyldimethoxysilane was changed to 19 parts.

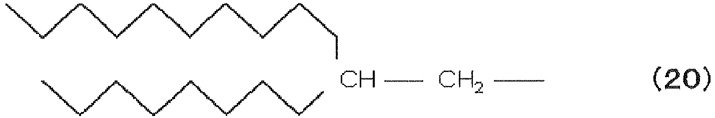

### Side chain amino-modified organopolysiloxane (a6) having long chain alkyl at both ends:

Side chain amino-modified organopolysiloxane (a6) having long chain alkyl at both ends with a nitrogen atom content of 0.91% and a viscosity of 1,300 mPa·s (measured with No. 3 rotor at 60 rpm) was obtained in the same manner as in the production method for (a1) except that the alkyl-modified polyorganosiloxane represented by the formula (19) was 46 parts, octamethylcyclotetrasiloxane was changed to 631 parts, and *N*-(2-aminoethyl)-3-aminopropylmethyldimethoxysilane was changed to 74 parts.

**Table 1**

| | R¹ | R² | R³ | x | y | N content (weight%) | Viscosity (mPa·s) 25°C |
|---|---|---|---|---|---|---|---|
| a1 | C₁₆₋₁₈H₃₃₋₃₇ (straight chain type) | CH₃ | *N*-(2-Aminoethyl)-3-aminopropyl group | 250 to 350 | 6 to 7 | 0.96 | 2100 |
| a2 | C₁₆₋₁₈H₃₃₋₃₇ (straight chain type) | CH₃ | *N*-(2-Aminoethyl)-3-aminopropyl group | 350 to 450 | 6 to 7 | 0.62 | 3500 |
| a3 | C₁₆₋₁₈H₃₃₋₃₇ (straight chain type) | CH₃ | *N*-(2-Aminoethyl)-3-aminopropyl group | 600 to 700 | 3 to 4 | 0.22 | 19000 |
| a4 | C₂₀₋₂₈H₄₁₋₅₇ (straight chain type) | CH₃ | *N*-(2-Aminoethyl)-3-aminopropyl group | 600 to 700 | 3 to 4 | 0.2 | 10000 |
| a7 | C₁₆₋₁₈H₃₃₋₃₇ (straight chain type) | CH₃ | *N*-(2-Aminoethyl)-3-aminopropyl group | 250 to 350 | 3 to 4 | 0.49 | 1200 |

**Table 2**

| | R¹ | R² | R³ | x | y | N content (weight%) | Viscosity (mPa·s) 25°C |
|---|---|---|---|---|---|---|---|
| a5 | C₂₀H₄₁ Guerbet branched type | CH₃ | *N*-(2-Aminoethyl)-3-aminopropyl group | 150 to 250 | 2 | 0.40 | 700 |
| a6 | C₂₀H₄₁ Guerbet branched type | CH₃ | *N*-(2-Aminoethyl)-3-aminopropyl group | 250 to 350 | 6 to 7 | 0.91 | 1300 |
| a3 | C₁₆₋₁₈H₃₃₋₃₇ (straight chain type) | CH₃ | *N*-(2-Aminoethyl)-3-aminopropyl group | 600 to 700 | 3 to 4 | 0.22 | 19000 |

In Tables 1 and 2, viscosity and nitrogen atom content were measured by the methods described in the above embodiment.

That is, the nitrogen atom content is the content of nitrogen atoms contained in the side chain amino-modified organopolysiloxane compound having long chain alkyl at both ends measured by neutralization titration.

A certain amount (0.5 to 10 g) of sample was dissolved in about 10 times the amount of a mixed solution (1:1 [vol%]) of isopropyl alcohol and toluene. Potassium tetrabromophenolphthalein ethyl ester was added thereto as an indicator, and neutralization titration was carried out using an aqueous solution of perchloric acid. The content of nitrogen atoms in the side chain amino-modified organopolysiloxane compound having long chain alkyl at both ends was calculated from the amount of sample taken and the normality and the amount consumed of the aqueous solution of perchloric acid.

Furthermore, with regard to the viscosity, a viscosity at 25°C of the side chain amino-modified organopolysiloxane compound having long chain alkyl at both ends was measured using a Vismetron viscometer (Shibaura Systems Co., Ltd.).

x and y were calculated from the amounts charged at the time of production.

### Examples 1 to 18 and Comparative Examples 1 to 11

Hair conditioners having the compositions shown in Table 3 and Table 4 were prepared by a standard method using any one of (a1) to (a6) as the side chain amino-modified organopolysiloxane having long chain alkyl at both ends (Component (A)), at least any one of *N,N*-dimethyl-3-octadecyloxypropylamine, octadecyloxy(2-hydroxypropyl)dimethylamine, and *N*-(3-(dimethylamino)propyl)docosanamide as Component (B), at least any one of stearyl alcohol and behenyl alcohol as Component (C), a polydimethylsiloxane as Component (D), water as Component (E), at least any one of benzyl alcohol and dipropylene glycol as Component (F), and lactic acid as Component (G). These hair conditioners were evaluated by the methods below in terms of performance when applying to damaged hair, during rinsing, and after drying. The results are given in Table 3 and Table 4.

The amount formulated of each component shown in Tables 3 and 4 is expressed as weight%.

### <Evaluation method>

Japanese female hair that had been subjected to one straight permanent treatment and two bleaching treatments was defined as damaged hair, and a bundle of hair weighing 20 g (length 20 cm, average diameter 80 µm) was subjected to sensory evaluation, while five panelists handled it, by the following method.

2 g of the hair conditioner shown in Table 3 and Table 4 was applied to a bundle of hair that had been washed using 3 g of a standard shampoo having the composition below, and after it was allowed to fully spread to the entire hair, evaluation during rinsing was carried out under running water at about 40°C for about 30 seconds. Subsequently, after it was dried with a towel and dried using a drier, evaluation after drying was carried out. Sensory evaluation was carried out in terms of smoothness when applying, smoothness during rinsing, suppleness during rinsing, and light slipperiness, suppleness, manageability, freedom from hair tangling, and freedom from stickiness after drying, and in some cases additional sensory evaluation was carried out in terms of shiny slipperiness when applying, shiny slipperiness during rinsing, and the presence/absence of luster, determination being made using the criteria below.

**Formulation of standard shampoo (pH 7.0)**

| | |
|---|---|
| 25% polyoxyethylene (2.5) lauryl ether sodium sulfate salt | 62.0% |
| Lauric acid diethanolamide | 2.3% |
| Disodium EDTA | 0.15% |
| Sodium benzoate | 0.5% |
| Sodium chloride | 0.8% |
| 75% phosphoric acid | appropriate amount |
| Fragrance, methylparaben | appropriate amount |
| Purified water | remainder |

### Evaluation criteria

5: 5 people answered that there was an effect
4: 4 people answered that there was an effect
3: 3 people answered that there was an effect
2: 2 people answered that there was an effect
1: 1 person answered that there was an effect
0: 0 people answered that there was an effect

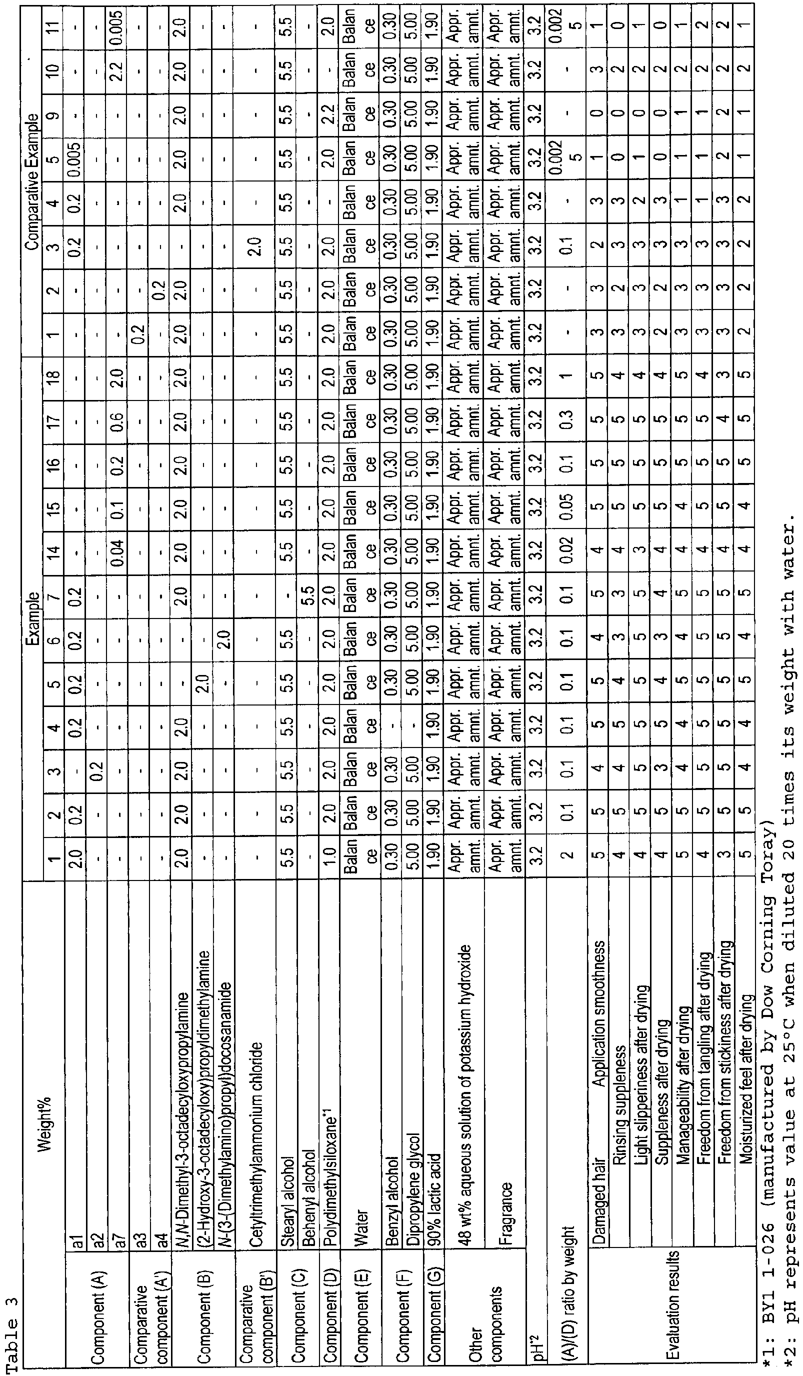

**Table 4**

| Weight% | | Example | | | | | | Comparative Example | | |
|---|---|---|---|---|---|---|---|---|---|---|
| | | 8 | 9 | 10 | 11 | 12 | 13 | 6 | 7 | 8 |
| Component (A) | a5 | 2.00 | 0.20 | - | 020 | 0.20 | 0.20 | - | - | 0.20 |
| | a6 | - | - | 0.20 | - | - | - | - | - | - |
| Comparative component (A') | a3 | - | - | - | - | - | - | - | 0.20 | - |
| Component (B) | *N,N*-Dimethyl-3-octadecyloxypropylamine | 2.00 | 2.00 | 2.00 | - | - | 2.00 | 2.00 | 2.00 | - |
| | (2-Hydroxy-3-octadecyloxy)propyldimethylamine | - | - | - | 2.00 | - | - | - | - | - |
| | *N*-(3-(Dimethylamino)propyl)docosanamide iii) | - | - | - | - | 2.00 | - | - | - | - |
| Comparative component (B') | Cetyltrimethylammonium chloride | - | - | - | - | - | - | - | - | 2.00 |
| Component (C) | Stearyl alcohol | 5.50 | 5.50 | 5.50 | 5.50 | 5.50 | - | 5.50 | 5.50 | 5.50 |
| | Behenyl alcohol | - | - | - | - | - | 5.50 | - | - | - |
| Component (D) | Polydimethylsiloxane^{*1} | 1.00 | 2.00 | 2.00 | 2.00 | 2.00 | 2.00 | 2.00 | 2.00 | 2.00 |
| Component (E) | Water | Balance | Balance | Balance | Balance | Balance | Balance | Balance | Balance | Balance |
| Component (F) | Benzyl alcohol | 0.30 | 0.30 | 0.30 | 0.30 | 0.30 | 0.30 | 0.30 | 0.30 | 0.30 |
| | Dipropylene glycol | 5.00 | 5.00 | 5.00 | 5.00 | 5.00 | 5.00 | 5.00 | 5.00 | 5.00 |
| Component (G) | 90% lactic acid | 1.90 | 1.90 | 1.90 | 1.90 | 1.90 | 1.90 | 1.90 | 1.90 | 1.90 |
| Other components | 48 wt% aqueous solution of potassium hydroxide | Appr. amount | Appr. amount | Appr. amount | Appr. amount | Appr. amount | Appr. amount | Appr. amount | Appr. amount | Appr. amount |
| | Fragrance | Appr. amount | Appr. amount | Appr. amount | Appr. amount | Appr. amount | Appr. amount | Appr. amount | Appr. amount | Appr. amount |
| pH^{*2} | | 3.2 | 3.2 | 3.2 | 3.2 | 3.2 | 3.2 | 3.2 | 3.2 | 3.2 |
| Evaluation results | Damaged hair Application smoothness | 5 | 5 | 3 | 5 | 5 | 5 | 0 | 3 | 2 |
| | Application shiny slipperiness | 4 | 5 | 4 | 5 | 5 | 5 | 0 | 2 | 2 |
| | Shiny slipperiness during rinsing | 4 | 5 | 4 | 5 | 5 | 5 | 0 | 2 | 3 |
| | Suppleness during rinsing | 4 | 5 | 3 | 3 | 4 | 4 | 0 | 3 | 2 |
| | Light slipperiness after drying | 4 | 5 | 4 | 5 | 3 | 3 | 0 | 3 | 3 |
| | Suppleness after drying | 4 | 5 | 3 | 3 | 4 | 3 | 0 | 2 | 2 |
| | Manageability after drying | 5 | 5 | 5 | 5 | 3 | 3 | 1 | 3 | 3 |
| | Freedom from tangling after drying | 4 | 5 | 4 | 5 | 3 | 3 | 1 | 3 | 3 |
| | Freedom from stickiness after drying | 4 | 5 | 4 | 5 | 5 | 4 | 2 | 3 | 3 |
| | Moisturized feel after drying | 5 | 5 | 4 | 5 | 4 | 5 | 1 | 2 | 2 |

| | | | | | | | | | | |
|---|---|---|---|---|---|---|---|---|---|---|
| *1: BY1 1-026 (manufactured by Dow Corning Toray) *2: pH represents a value at 25°C when diluted 20 times its weight with water. | | | | | | | | | | |

### Example 19

A hair conditioner having the composition below was prepared. When this hair conditioner was used, even damaged hair exhibited light slipperiness when applying and during rinsing, light slipperiness after drying, suppleness, manageability, freedom from hair tangling, and freedom from stickiness.

**<Composition>**

| | |
|---|---|
| (a1) | 0.20% |
| *N,N*-Dimethyl-3-octadecyloxypropylamine | 1.50% |
| Lactic acid | 1.00% |
| Malic acid | 0.20% |
| Stearyl alcohol | 5.50% |
| Benzyl alcohol | 0.50% |
| Dipropylene glycol | 2.50% |
| Cholesteryl isostearate | 0.10% |
| Hydroxyethylcellulose^{*2} | 0.10% |
| Dimethylpolysiloxane (polydimethylsiloxane)^{*3} | 2.00% |
| Dimethylpolysiloxane (polydimethylsiloxane)^{*4} | 2.00% |
| Amino-modified silicone*⁵ | 0.50% |
| Fragrance | appropriate amount |
| 48 weight% aqueous solution of potassium hydroxide | appropriate amount |
| Purified water (pH 3.3) | remainder |

| | |
|---|---|
| *2 HEC Daicel SE850K manufactured by Daicel Chemical Industries, Ltd. *3 BY11-040 manufactured by Dow Corning Toray *4 BY11-026 manufactured by Dow Corning Toray *5 SM8904 COSMETIC EMULSION manufactured by Dow Corning Toray | |

### Example 20

A hair conditioner with an antidandruff effect having the composition below was prepared. When this hair conditioner was used, even damaged hair exhibited light slipperiness when applying and during rinsing, light slipperiness after drying, suppleness, manageability, freedom from hair tangling, and freedom from stickiness. Furthermore, an anti-dandruff effect could be confirmed.

**<Composition>**

| | |
|---|---|
| (a1) | 0.20% |
| *N,N*-Dimethyl-3-octadecyloxypropylamine | 1.50% |
| Lactic acid | 1.00% |
| Malic acid | 0.50% |
| Cetyl alcohol | 4.00% |
| Dipotassium glycyrrhizinate^{*2} | 0.10% |
| Benzyloxyethanol | 1.00% |
| Isopropyl palmitate | 0.50% |
| Hybrid sunflower oil | 0.50% |
| Hydroxyethylcellulose^{*3} | 0.30% |
| Dimethylpolysiloxane (polydimethylsiloxane)^{*4} | 3.00% |
| Amino-modified silicone^{*5} | 0.20% |
| Fragrance | appropriate amount |
| 48 weight% aqueous solution of potassium hydroxide | appropriate amount |
| Purified water (pH 3.7) | remainder |

| | |
|---|---|
| *2 Glytinon K2 manufactured by Tokiwa Phytochemical Co., Ltd. *3 HEC Daicel SE850K manufactured by Daicel Chemical Industries, Ltd. *4 BY11-040 manufactured by Dow Corning Toray *5 SM8904 COSMETIC EMULSION manufactured by Dow Corning Toray | |

### Example 21

A hair treatment having the composition below was prepared. When this hair treatment was used, even damaged hair exhibited light slipperiness when applying and during rinsing, light slipperiness after drying, suppleness, manageability, freedom from hair tangling, and freedom from stickiness.

**<Composition>**

| | |
|---|---|
| (a2) | 0.50% |
| *N,N*-Dimethyl-3-octadecyloxypropylamine | 2.50% |
| Lactic acid | 1.20% |
| Malic acid | 0.20% |
| Stearyl alcohol | 8.00% |
| Benzyl alcohol | 1.00% |
| Dipropylene glycol | 5.00% |
| 18-Methyleicosanoic acid^{*2} | 0.10% |
| Dipentaerythritol fatty acid ester | 0.30% |
| Polypropylene glycol*3 | 0.20% |
| Dimethylpolysiloxane (polydimethylsiloxane)^{*4} | 3.00% |
| Dimethylpolysiloxane (polydimethylsiloxane)^{*5} | 2.00% |
| Amino-modified silicone^{*6} | 0.50% |
| (Bisisobutyl PEG-15/Amodimethicone) copolymer^{*7} | 0.20% |
| Fragrance | appropriate amount |
| 48 weight% aqueous solution of potassium hydroxide | appropriate amount |
| Purified water (pH 3.3) | remainder |

| | |
|---|---|
| *2 Crodacid 18MEA, manufactured by Croda Japan *3 Polyox WSR *N*-60K, manufactured by The Dow Chemical Company *4 BY11-040, manufactured by Dow Corning Toray *5 BY11-026, manufactured by Dow Corning Toray *6 SM8904 COSMETIC EMULSION, manufactured by Dow Corning Toray *7 Dow Corning Toray SS-3588, manufactured by DOW CORNING TORAY | |

### Example 22

A hair treatment having the composition below was prepared. When this hair treatment was used, even damaged hair exhibited light slipperiness when applying and during rinsing, shiny slipperiness, light slipperiness after drying, suppleness, manageability, freedom from hair tangling, freedom from stickiness, and luster. Furthermore, it was confirmed that the hair was free from stickiness.

**<Composition>**

| | |
|---|---|
| (a6) | 0.50% |
| *N,N*-Dimethyl-3-octadecyloxypropylamine | 2.50% |
| Lactic acid | 1.20% |
| Malic acid | 0.20% |
| Stearyl alcohol | 8.00% |
| Benzyl alcohol | 1.00% |
| Dipropylene glycol | 5.00% |
| 18-Methyleicosanoic acid^{*2} | 0.10% |
| Dipentaerythritol fatty acid ester | 0.30% |
| Polypropylene glycol^{*3} | 0.20% |
| Dimethylpolysiloxane (polydimethylsiloxane)^{*4} | 3.00% |
| Dimethylpolysiloxane (polydimethylsiloxane)^{*5} | 2.00% |
| Amino-modified silicone^{*6} | 0.50% |
| (Bisisobutyl PEG-15/Amodimethicone) copolymer^{*7} | 0.20% |
| Fragrance | appropriate amount |
| 48 weight% aqueous solution of potassium hydroxide | appropriate amount |
| Purified water (pH 3.3) | remainder |

| | |
|---|---|
| *2 Crodacid 18MEA manufactured by Croda Japan *3 Polyox WSR *N*-60K manufactured by The Dow Chemical Company *4 BY11-040 manufactured by Dow Corning Toray *5 BY11-026 manufactured by Dow Corning Toray *6 SM8904 COSMETIC EMULSION manufactured by Dow Corning Toray *7 DOW CORNING TORAY SS-3588 manufactured by Dow Corning Toray | |

## Claims

1. A hair cosmetic comprising the following components (A) to (E):
(A) 0.01 to 15 weight% of a side chain amino-modified organopolysiloxane having long chain alkyl at both ends, represented by the following formula (1):
(R¹R²₂)SiO-(R²₂SiO)ₓ-(R²R³SiO)_{y}-Si(R¹R²₂) (1)
wherein, in the above formula (1), R¹ represents a straight chain or branched alkyl group having 12 to 50 carbon atoms, R² represents a substituted or unsubstituted monovalent hydrocarbon group having 1 to 6 carbon atoms, R³ represents a 3-aminopropyl group or an *N*-(2-aminoethyl)-3-aminopropyl group, x is 1 to 2000, and y is an integer of 1 to 100;
(B) 0.1 to 15 weight% of one type or two or more types of cationic surfactants selected from tertiary amine compounds;
(C) 0.1 to 20 weight% of a higher alcohol having 12 to 28 carbon atoms and one hydroxy group;
(D) 0.01 to 15 weight% of a polydimethylsiloxane; and
(E) water,
wherein a nitrogen atom content of the side chain amino-modified organopolysiloxane having long chain alkyl at both ends is 0.31 to 2 weight%, and
wherein a weight ratio of the component (A) to the component (D), (A)/(D), is 0.01 to 2.

2. The hair cosmetic according to Claim 1, wherein in the formula (1) of the component (A), the ratio of y to x, y/x, is 0.003 to 0.1.

3. The hair cosmetic according to Claims 1 or 2, wherein in the formula (1) of the component (A), R¹ is a straight chain alkyl group having 12 to 50 carbon atoms.

4. The hair cosmetic according to Claims 1 or 2, wherein in the formula (1) of the component (A), R¹ is a branched alkyl group having 12 to 50 carbon atoms.

5. The hair cosmetic according to any one of Claims 1 to 4, wherein the component (B) is at least one of a hydroxy ether alkylamine and a salt thereof, an ether amine and a salt thereof, and an alkylamidoamine and a salt thereof.

6. The hair cosmetic according to any one of Claims 1 to 5, wherein it further comprises the following component (F):
(F) an organic solvent selected from the following (f1) to (f6)
(f1) a compound represented by the following formula (2): 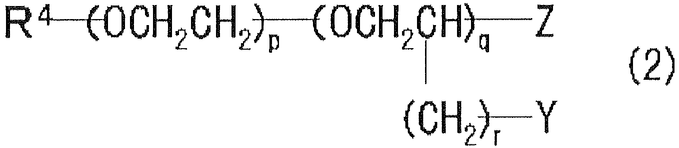 wherein, in the above formula (2), R⁴ represents a hydrogen atom, an alkyl group having 1 to 4 carbon atoms, or the group R²⁶-Ph-R²⁷ (R²⁶: a hydrogen atom, a methyl group, or a methoxy group, R²⁷: a direct bond or a saturated or unsaturated divalent hydrocarbon group having 1 to 3 carbon atoms, Ph: phenylene group), Y and Z independently represent a hydrogen atom or a hydroxy group, and p, q, and r independently represent an integer of 0 to 5, provided that when p = q = 0, R⁴ is neither a hydrogen atom nor the group R²⁶-Ph-R²⁷ and Z is a hydroxy group;
(f2) an *N*-alkylpyrrolidone in which an alkyl group having 1 to 18 carbon atoms is bonded to the nitrogen atom;
(f3) an alkylene carbonate having an alkylene group having 2 to 4 carbon atoms;
(f4) a polypropylene glycol having a molecular weight of 100 to 5000;
(f5) a lactone or cyclic ketone represented by the following formula (3), (4), or (5): 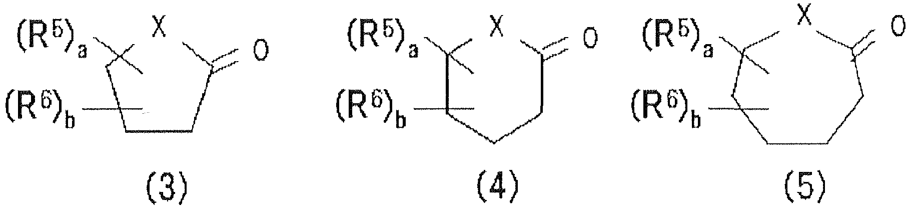 wherein, in the above formulae (3) to (5), X represents a methylene group or an oxygen atom, R⁵ and R⁶ represent mutually different substituents, and a and b independently represent 0 or 1; and
(f6) a diol represented by the following formula (6): 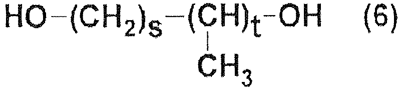 wherein, in the above formula (6), s and t independently represent an integer of 0 to 6, provided that when s = 0, t is 1 or more, and when t = 0, s is 1 or more.

7. The hair cosmetic according to any one of Claims 1 to 6, which comprises (G) an organic acid.

8. The hair cosmetic according to any one of Claims 1 to 7, which has a pH of 2 to 5 when diluted to 20 times the weight of the composition with water.

## Patentansprüche

1. Haarkosmetikum, enthaltend die folgenden Komponenten (A) bis (D):
(A) 0,01 bis 15 Gew.% eines Amino-modifizierten Seitenketten-Organopolysiloxans mit langkettigem Alkyl an beiden Enden, dargestellt durch die folgende Formel (1):
(R¹R²₂)SiO-(R²₂SiO)ₓ-(R²R³SiO)_{y}-Si(R¹R²₂) (1)
worin in der obigen Formel (1) R¹ eine geradkettige oder verzweigte Alkylgruppe mit 12 bis 50 Kohlenstoffatomen ist, R² eine substituierte oder unsubstituierte monovalente Kohlenwasserstoffgruppe mit 1 bis 6 Kohlenstoffatomen ist, R³ eine 3-Aminopropylgruppe oder eine N-(2-Aminoethyl)-3-aminopropylgruppe ist, x 1 bis 2.000 ist und y eine ganze Zahl von 1 bis 100 ist;
(B) 0,1 bis 15 Gew.% von einem oder zwei oder mehreren Typen von kationischen Tensiden, ausgewählt aus tertiären Aminverbindungen;
(C) 0,1 bis 20 Gew.% eines höheren Alkohols mit 12 bis 28 Kohlenstoffatomen und einer Hydroxygruppe;
(D) 0,01 bis 15 Gew.% eines Polydimethylsiloxans; und
(E) Wasser,
worin ein Stickstoffatomgehalt des Amino-modifizierten Seitenketten-Organopolysiloxans mit langkettigem Alkyl an beiden Enden 0,31 bis 2 Gew.% ist, und
worin ein Gewichtsverhältnis der Komponente (A) zu der Komponente (D), (A)/(D), 0,01 bis 2 ist.

2. Haarkosmetikum gemäß Anspruch 1, worin in der Formel (1) der Komponente (A) das Verhältnis von y zu x, y/x, 0,003 bis 0,1 ist.

3. Haarkosmetikum gemäß Anspruch 1 oder 2, worin in der Formel (1) der Komponente (A) R¹ eine geradkettige Alkylgruppe mit 12 bis 50 Kohlenstoffatomen ist.

4. Haarkosmetikum gemäß Anspruch 1 oder 2, worin in der Formel (1) der Komponente (A) R¹ eine verzweigte Alkylgruppe mit 12 bis 50 Kohlenstoffatomen ist.

5. Haarkosmetikum gemäß einem der Ansprüche 1 bis 4, worin die Komponente (B) zumindest eines von einem Hydroxyetheralkylamin und einem Salz davon, Etheramin und einem Salz davon und Alkylamidoamin und einem Salz davon ist.

6. Haarkosmetikum gemäß einem der Ansprüche 1 bis 5, das weiterhin die folgende Komponente (F) enthält:
(F) ein organisches Lösungsmittel, ausgewählt aus dem Folgenden (f1) bis (f6)
(f1) Verbindung mit der folgenden Formel (2): 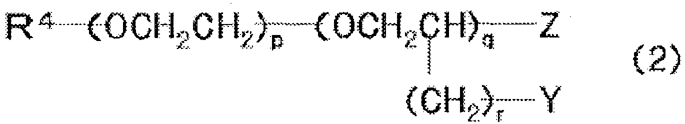 worin in der obigen Formel (2) R⁴ ein Wasserstoffatom, eine Alkylgruppe mit 1 bis 4 Kohlenstoffatomen oder die Gruppe R²⁶-Ph-R²⁷ ist (R²⁶: Wasserstoffatom, Methylgruppe oder Methoxygruppe, R²⁷: direkte Bindung oder gesättigte oder ungesättigte divalente Kohlenwasserstoffgruppe mit 1 bis 3 Kohlenstoffatomen, Ph: Phenylengruppe) Y und Z unabhängig ein Wasserstoffatom oder eine Hydroxygruppe sind und p, q und r unabhängig eine ganze Zahl von 0 bis 5 sind, vorausgesetzt, dass dann, wenn p = q = 0, R⁴ weder ein Wasserstoffatom noch die Gruppe R²⁶-Ph-R²⁷ ist und Z eine Hydroxygruppe ist,
(f2) N-Alkylpyrrolidon, worin eine Alkylgruppe mit 1 bis 18 Kohlenstoffatomen an das Stickstoffatom gebunden ist,
(f3) Alkylencarbonat mit einer Alkylengruppe mit 2 bis 4 Kohlenstoffatomen,
(f4) Polypropylenglykol mit einem Molekulargewicht von 100 bis 5.000,
(f5) Lakton oder cyclisches Keton mit der folgenden Formel (3), (4) oder (5): 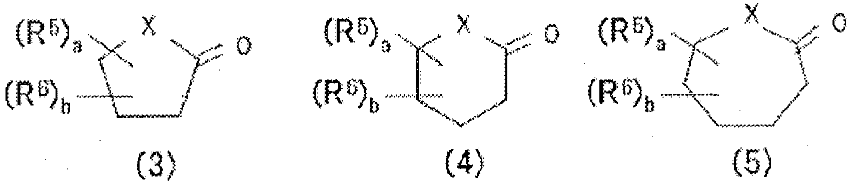 worin in den obigen Formeln (3) bis (5) X eine Methylengruppe oder ein Sauerstoffatom ist, R⁵ und R⁶ wechselseitig verschiedene Substituenten sind und a und b unabhängig 0 oder 1 ist, und
(f6) Diol mit der folgenden Formel (6): 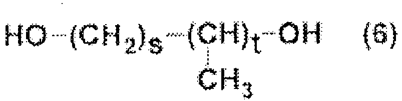 worin in der obigen Formel (6) s und t unabhängig eine ganze Zahl von 0 bis 6 sind, vorausgesetzt, dass dann, wenn s = 0, t 1 oder mehr ist und wenn t = 0, s 1 oder mehr ist.

7. Haarkosmetikum gemäß einem der Ansprüche 1 bis 6, umfassend (G) eine organische Säure.

8. Haarkosmetikum gemäß einem der Ansprüche 1 bis 7, das einen pH von 2 bis 5 hat, wenn auf das 20-fache des Gewichtes der Zusammensetzung mit Wasser verdünnt ist.

## Revendications

1. Produit cosmétique capillaire comprenant les composants (A) à (E) suivants :
(A) 0,01 à 15% en poids d'un organopolysiloxane amino modifié à chaîne latérale ayant un alkyle à longue chaîne aux deux extrémités, représenté par la formule (1) suivante :
(R¹R²₂)SiO-(R²₂SiO)ₓ-(R²R³SiO)_{y}-Si(R¹R²₂) (1)
dans lequel, dans la formule (1) ci-dessus, R¹ représente un groupe alkyle à chaîne linéaire ou ramifiée ayant 12 à 50 atomes de carbone, R² représente un groupe hydrocarboné monovalent substitué ou non substitué ayant 1 à 6 atomes de carbone, R³ représente un groupe 3-aminopropyle ou un groupe N-(2-aminoéthyl)-3-aminopropyle, x représente 1 à 2000, et y est un nombre entier de 1 à 100;
(B) 0,1 à 15 % en poids d'un type ou deux ou plus de deux types de tensioactifs cationiques choisis parmi des composés d'amine tertiaire ;
(C) 0,1 à 20 % en poids d'un alcool supérieur ayant 12 à 28 atomes de carbone et un groupe hydroxy ;
(D) 0,01 à 15 % en poids d'un polydiméthylsiloxane ; et
(E) de l'eau,
dans lequel une teneur en atomes d'azote de l'organopolysiloxane amino modifié à chaîne latérale ayant un alkyle à longue chaîne aux deux extrémités est de 0,31 à 2 % en poids, et
dans lequel un ratio pondéral du composant (A) par rapport au composant (D), (A)/(D), est 0,01 à 2.

2. Produit cosmétique capillaire selon la revendication 1, dans lequel dans la formule (1) du composant (A), le ratio de y par rapport à x, y/x, est de 0,003 à 0,1.

3. Produit cosmétique capillaire selon la revendication 1 ou 2, dans lequel dans la formule (1) du composant (A), R¹ est un groupe alkyle à chaîne linéaire ayant 12 à 50 atomes de carbone.

4. Produit cosmétique capillaire selon la revendication 1 ou 2, dans lequel dans la formule (1) du composant (A), R¹ est un groupe alkyle ramifié ayant 12 à 50 atomes de carbone.

5. Produit cosmétique capillaire selon l'une quelconque des revendications 1 à 4, dans lequel le composant (B) est au moins l'un parmi une hydroxy éther alkylamine et un sel de celle-ci, une éther amine et un sel de celle-ci et une alkylamidoamine et un de celle-ci.

6. Produit cosmétique capillaire selon l'une quelconque des revendications 1 à 5, dans lequel il comprend en outre le composant (F) suivant :
(F) un solvant organique choisi parmi les suivants (f1) à (f6)
(f1) un composé représenté par la formule suivante (2) 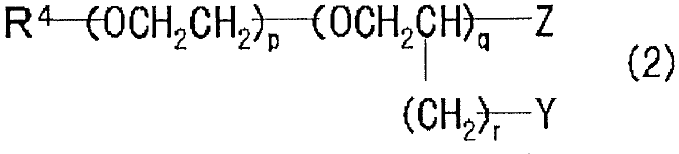 dans lequel, dans la formule (2) ci-dessus, R⁴ représente un atome d'hydrogène, un groupe alkyle ayant 1 à 4 atomes de carbone ou le groupe R²⁶-Ph-R²⁷ (R²⁶: un atome d'hydrogène, un groupe méthyle ou un groupe méthoxy, R²⁷: une liaison directe ou un groupe hydrocarboné divalent saturé ou insaturé ayant 1 à 3 atomes de carbone, Ph : groupe phénylène), Y et Z représentent indépendamment un atome d'hydrogène ou un groupe hydroxy, et p, q, et r représentent indépendamment un nombre entier de 0 à 5, à condition que lorsque p = q = 0, R⁴ n'est ni un atome d'hydrogène ni le groupe R²⁶-Ph-R²⁷ et Z est un groupe hydroxy ;
(f2) une *N*-alkylpyrrolidone dans laquelle un groupe alkyle ayant 1 à 18 atomes de carbone est lié à l'atome d'azote;
(f3) un carbonate d'alkylène ayant un groupe alkylène ayant 2 à 4 atomes de carbone ;
(f4) un polypropylène glycol ayant un poids moléculaire de 100 à 5000;
(f5) une lactone ou cétone cyclique représentée par la formule suivante (3), (4) ou (5) : 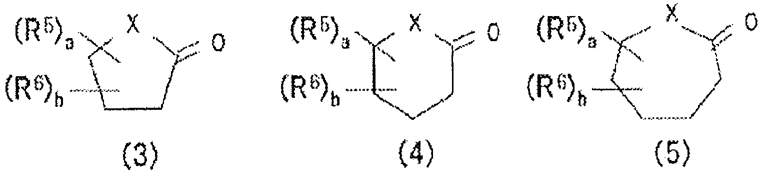 dans lequel, dans les formules (3) à (5) ci-dessus, X représente un groupe méthylène ou un atome d'oxygène, R⁵ et R⁶ représentent des substituants mutuellement différents, et a et b représentent indépendamment 0 ou 1 ; et
(f6) un diol représenté par la formule (6) suivante : 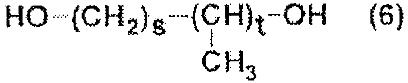 dans lequel, dans la formule (6) ci-dessus, s et t représentent indépendamment un nombre entier de 0 à 6, à condition que lorsque s = 0, t est égal à 1 ou plus, et quand t = 0, s est égal à 1 ou plus.

7. Produit cosmétique capillaire selon l'une quelconque des revendications 1 à 6, qui comprend (G) un acide organique.

8. Produit cosmétique capillaire selon l'une quelconque des revendications 1 à 7, qui a un pH de 2 à 5 lorsqu'il est dilué avec de l'eau à 20 fois par rapport au poids de la composition.
